# EUROPÄISCHE PATENTSCHRIFT

(11) **EP 1 864 648 B1**
(45) Veröffentlichungstag und Bekanntmachung des Hinweises auf die Patenterteilung: **16.11.2011**
(21) Anmeldenummer: 07109386.8
(22) Anmeldetag: 01.06.2007
(51) Int. Cl.: A61K 8/41, A61Q 17/00

(54) **Kosmetische Zubereitung mit acyclischem Merocyanin**
Acyclical merocyanines in cosmetic preparations
Mérocyanine acyclique dans des préparations cosmétiques

(30) Priorität: 09.06.2006 DE 102006027257
(43) Veröffentlichungstag der Anmeldung: 12.12.2007
(73) Patentinhaber: Beiersdorf AG, 20253 Hamburg (DE)
(72) Erfinder: Mundt, Claudia, 28207, Bremen (DE); Skubsch, Kerstin, 25421, Pinneberg (DE); Reinecke, Myriam, 22529, Hamburg (DE); Wolber, Rainer, 22397, Hamburg (DE); Blatt, Thomas, 21379, Wedel (DE); Smuda, Christoph, 25474, Bönningstedt (DE)

(56) Entgegenhaltungen:
- WO-A-2004/006878
- WO-A-2006/003094
- WO-A-2007/071584

## Beschreibung

Die vorliegende Erfindung betrifft eine kosmetische Zubereitung mit einem acyclischen Merocyanin.

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Zum Schutz der Haut wurden daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie dem Anlage 7 der Kosmetikverordnung zusammengefasst.

Die Vielzahl an kommerziell erhältlichen Sonnenschutzmitteln darf jedoch nicht darüber hinwegtäuschen, dass diese Zubereitungen des Standes der Technik eine Reihe von Nachteilen aufweisen.

Kosmetische Zubereitungen, die wie Sonnenschutzzubereitungen auf die Haut aufgetragen werden, kommen regelmäßig (beabsichtigt oder unbeabsichtigt) mit Kleidungsstücken in Kontakt, an denen sie (z.B. als "Abrieb" oder weil sie von den Faserstoffen "aufgesaugt" werden) teilweise haften bleiben. Insbesondere wenn die kosmetischen Zubereitungen eine leichte Eigenfarbe aufweisen oder einen gewissen Anteil an lipophilen Komponenten enthalten (wie es bei nahezu jeder Emulsion oder Dispersion der Fall ist) führt dies zu einer optisch wahrnehmbaren Verunreinigung (z.B. Verfärbung) des Kleidungsstückes. Besonders auffällig ist dieser Effekt bei weißen Kleidungsstücken. Wenn beispielsweise in Vorbereitung auf ein Sonnenbad schon "zu Hause" das Sonnenschutzmittel auf die Haut aufgetragen wird und auf dem Weg zum "Badeort" eine leichte Sommerbekleidung (T-Shirts, Hemden, Röcke, Shorts) getragen wird, kommen diese Textilien unmittelbar mit der frisch applizierten Lichtschutzzubereitung in Kontakt und nehmen mehr oder weniger große Mengen davon auf. Ebenso häufig kommt es nach Beendigung des Sonnenbades zu einem Transfer von "Kosmetik-Rückständen" (Überresten des Sonnenschutzmittels) auf die dann vom Sonnenanbeter wieder angezogene Kleidung. Besonders intensiv ist regelmäßig der Kontakt von Badebekleidung (z.B. Bikinis, Badehosen, Badeanzügen, Handtüchern, Badelaken) und Sonnenschutzmitteln.

Nachteilig am Stande der Technik ist dabei insbesondere der Umstand, dass die einmal von den Textilien aufgenommenen Kosmetika (z.B. Sonnenschutzmittel) sich nur relativ schwer wieder auswaschen lassen. Regelmäßig bleiben mehr oder weniger große Reste des Kosmetikums auch nach einem Waschgang noch auf der Kleidung zurück. Nach dem Stand der Technik bleibt dem Verbraucher häufig nur die Möglichkeit die Kleidungsstücke wiederholt zu waschen, bei höherer Temperatur zu waschen und/oder größere Mengen an Waschmittel aufzuwenden. All diese Maßnahmen führen zu einer erhöhten Umweltbelastung und zu einem erhöhten Verschleiß der Textilien, die dadurch häufiger ersetzt werden müssen.

Es war daher die Aufgabe der vorliegenden Erfindung diesen Nachteil des Standes der Technik zu beseitigen. Insbesondere war es die Aufgabe der vorliegenden Erfindung, kosmetische Zubereitungen (insbesondere Sonnenschutzmittel) zu entwickeln, welche die Haut, Haare und Nägel zuverlässig vor den negativen Auswirkungen des Sonnenlichtes schützen und sich dabei besonders leicht und für das menschliche Auge sichtbar aus den mit diesen Zubereitungen verunreinigten Kleidungsstücken (inklusive Halstücher) wieder auswaschen lassen.

Dabei sollte die Zubereitung die Haut insbesondere vor den schädlichen Auswirkungen der UV-A-Strahlung wirksam schützen.

Erfindungsgemäß wird die Aufgabe gelöst durch eine kosmetische Zubereitung enthaltend die Verbindung:

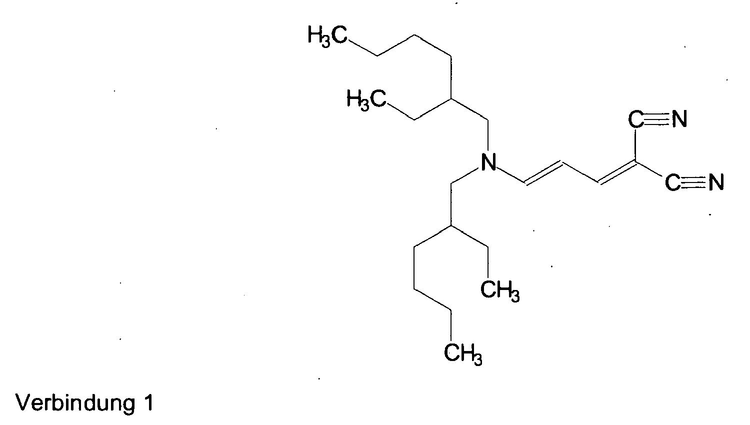

Die erfindungsgemäße Lösung war insbesondere deshalb überraschend, da es sich bei der erfindungsgemäßen Verbindung 1 um eine nahezu wasserunlösliche Verbindung handelt.

Die erfindungsgemäßen Zubereitungen zeichnen sich auch überraschend dadurch aus, dass Sie die Haut, bzw. Haar und/oder Nägel beim Auftragen zunächst leicht färben, so dass der Anwender bei der Applikation visuell nachvollziehen kann welche Körperpartien er in welchem Umfang mit der Zubereitung behandelt hat. Es können dadurch insbesondere Zubereitungen dargereicht werden, bei denen der UVA-Schutz und insbesondere der UVA-1-Schutz der Haut visuell durch den Verbraucher nachvollzogen werden kann.

Die erfindungsgemäßen Zubereitungen zeigen darüber hinaus eine erhöhte Photostabilität. Insbesondere Zubereitungen, welche photolabile Inhaltstoffe wie 4-(tert.-Butyl)-4'-methoxydibenzoylmethan enthalten, zeigen bei der Bestrahlung über einen längeren Zeitraum einen deutlich geringeren Abfall der Lichtschutzfilterwirkung als Zubereitungen ohne den erfindungsgemäßen Lichtschutzfilter.

Die Kombination mit weiteren Lichtschutzfiltern führt im Regelfall zu einem überadditiven Anstieg des Lichtschutzfaktors (SPF) und zu einer hohen UV-A-Balance. Hautpartien, die mit der erfindungsgemäßen kosmetischen Zubereitung behandelt wurden, zeigen nach der Bestrahlung mit UV-Strahlen eine deutlich höhere Vitalität (Zellvitalität), als Hautpartien, die mit vergleichbaren Zubereitungen behandelt wurden, welche den erfindungsgemäßen Lichtschutzfilter nicht enthielten.

Unpolare organische Lichtschutzfilter wie beispielsweise Benzoylmethanderivate oder Triazine neigen in kosmetischen Zubereitungen regelmäßig zum Auskristallisieren, was nur mit hohem formulierungstechnischen Aufwand halbwegs unterdrückt werden kann. Der erfindungsgemäße Lichtschutzfilter hingegen zeigt im Vergleich dazu überraschenderweise eine deutlich geringere Neigung zur Auskristallisierung.

Es ist erfindungsgemäß bevorzugt, wenn die erfindungsgemäßen Zubereitungen die Verbindung 1 in einer Konzentration von 10 bis 0,1 Gewichts-%, und besonders bevorzugt in einer Konzentration von 5 bis 0,5 Gewichts-%, jeweils bezogen auf das Gesamtgewicht der Zubereitung, enthalten.

Es ist erfindungsgemäß vorteilhaft, wenn die erfindungsgemäße Zubereitung in Form eines Gels, einer Emulsion, einer Dispersion oder eines Öls vorliegt. Dabei sind Emulsionen und Dispersionen erfindungsgemäß bevorzugt.

Es ist erfindungsgemäß besonders bevorzugt, wenn die erfindungsgemäße Zubereitung in Form einer O/W-Emulsion vorliegt.

Liegt die erfindungsgemäße Zubereitung in Form einer O/W-Emulsion vor, so ist sie erfindungsgemäß bevorzugt **dadurch gekennzeichnet, dass** die Zubereitung einen oder mehrere O/W-Emulgatoren gewählt aus der Gruppe der Verbindungen Glycerylstearatcitrat, Glycerylstearat (selbstemulgierend), Stearinsäure, Stearatsalze, Polyglyceryl-3-methylglycosedistearat, Ceteareth-20, PEG-40 Stearat , Natriumcetearylsulfat enthält. Ferner ist es vorteilhaft im Sinne der vorliegenden Erfindung Cetearylalkohol in Kombination mit PEG-40 hydriertes Rizinusöl, Natriumcetearylsulfat und Glycerylstearat. Ausserdem ist es erfindungsgemäß vorteilhaft Kaliumcetylphosphat als Emulgator einzusetzen.

Diese erfindungsgemäßen O/W-Emulgatoren können erfindungsgemäß vorteilhaft in einer Konzentration von 0,001 bis 10 Gewichts-% und bevorzugt in einer Konzentration von 0,1 bis 7 Gewichts-%, bezogen auf das Gesamtgewicht der Zubereitung in dieser enthalten sein.

In einer weiteren erfindungsgemäß besonders bevorzugten Ausführungsform liegt die erfindungsgemäße Zubereitung in Form einer W/O-Emulsion vor.

In dieser Ausführungsform ist es erfindungsgemäß besonders bevorzugt, wenn die Zubereitung einen oder mehrere W/O-Emulgatoren gewählt aus der Gruppe der Verbindungen Polyglyceryl-2-dipolyhydroxystearat, PEG-30 Dipolyhydroxystearat, Cetyl Dimethicon Copolyol, Polyglyceryl-3 Diisostearat enthält.

Diese erfindungsgemäßen W/O-Emulgatoren können erfindungsgemäß vorteilhaft in einer Konzentration von 0,1 bis 10 Gewichts-% und bevorzugt in einer Konzentration von 0,2 bis 7 Gewichts-%, bezogen auf das Gesamtgewicht der Zubereitung in dieser enthalten sein.

Erfindungsgemäß vorteilhafte Ausführungsformen der vorliegenden Erfindung sind **dadurch gekennzeichnet, dass** die Zubereitung einen oder mehrere weitere UV-Filter enthält, gewählt aus der Gruppe der Verbindungen Phenylen-1,4-bis-(2-benzimidazyl)-3,3'-5,5'-tetrasulfonsäuresalze; 2-Phenylbenzimidazol-5-sulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol) (Handelsname: Tinosorb M); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat; Terephthalidendicamphersulfonsäure; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 2-(4'-Diethylamino-2'-hydoxybenzoyl)-benzoesäurehexylester, 4-(tert.-Butyl)-4'-methoxydibenzoylmethan; Homomenthylsalicylat; 2-Ethylhexyl-2-hydroxybenzoat ist doch = Ethylhexylsalicylat, oder; 2-Ethylhexyl-2-cyano-3,3-diphenylacrylat; Dimethicodiethylbenzalmalonat; 3-(4-(2,2-bis Ethoxycarbonylvinyl)-phenoxy)propenyl)-methoxysiloxan /Dimethylsiloxan - Copolymer (INCI: Polysilicone-15); 2,4-Bis-{[4-(2-ethyl-hexyloxy)-2-hydroxy]-phenyl}-6-(4-methoxyphenyl)-1,3,5-triazin (INCI :Bis-Ethylhexyloxyphenol Methoxyphenyl Triazin); Dioctylbutylamidotriazon (INCI: Diethylhexyl-Butamidotriazone); 2,4-bis-[5-1(dimethylpropyl)benzoxazol-2-yl-(4-phehyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoësäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4,6-Tribiphenyl-4-yl-1,3,5-triazin; den Merocyaninverbindungen 1 bis 4, welche in der DE102005059741.6 offenbart sind;Titiandioxid; Zinkoxid in einer Konzentration von 0,01 bis 40 Gewichts-% bezogen auf das Gesamtgewicht der Zubereitung. Dabei sind Konzentrationen von 1 bis 20 Gewichts-%, bezogen auf das Gesamtgewicht der Zubereitung erfindungsgemäß bevorzugt.

Es ist erfindungsgemäß vorteilhaft, wenn die erfindungsgemäße Zubereitung frei ist von p-Methylbenzylidencampher.

Die Pigmente (Titandioxid, Zinkoxid) können vorteilhaft im Sinne der vorliegenden Erfindung auch in Form kommerziell erhältlicher öliger oder wäßriger Vordispersionen zur Anwendung kommen. Diesen Vordispersionen können vorteilhaft Dispergierhilfsmittel und/oder Solubilisationsvermittler zugesetzt sein.

Die Pigmente (Titandioxid, Zinkoxid) können erfindungsgemäß vorteilhaft oberflächlich behandelt ("gecoatet") sein, wobei beispielsweise ein hydrophiler, amphiphiler oder hydrophober Charakter gebildet werden bzw. erhalten bleiben soll. Diese Oberflächenbehandlung kann darin bestehen, dass die Pigmente nach an sich bekannten Verfahren mit einer dünnen hydrophilen und/oder hydrophoben anorganischen und/oder organischen Schicht versehen werden. Die verschiedenen Oberflächenbeschichtungen können im Sinne der vorliegenden Erfindung auch Wasser enthalten.

Anorganische Oberflächenbeschichtungen im Sinne der vorliegenden Erfindung können bestehen aus Aluminiumoxid (Al₂O₃), Aluminiumhydroxid Al(OH)₃, bzw. Aluminiumoxidhydrat (auch: Alumina, CAS-Nr.: 1333-84-2), Natriumhexametaphosphat (NaPO₃)₆, Natriummetaphosphat (NaPO₃)ₙ, Siliciumdioxid (SiO₂) (auch: Silica, CAS-Nr.: 7631-86-9), Bariumsulfat (Ba-SO₄) oder Eisenoxid (Fe₂O₃). Diese anorganischen Oberflächenbeschichtungen können allein, in Kombination und/oder in Kombination mit organischen Beschichtungsmaterialien vorkommen.

Organische Oberflächenbeschichtungen im Sinne der vorliegenden Erfindung können bestehen aus pflanzlichem oder tierischem Aluminiumstearat, pflanzlicher oder tierischer Stearinsäure, Laurinsäure, Dimethylpolysiloxan (auch: Dimethicone), Methylpolysiloxan (Methicone), Simethicone (einem Gemisch aus Dimethylpolysiloxan mit einer durchschnittlichen Kettenlänge von 200 bis 350 Dimethylsiloxan-Einheiten und Silicagel) oder Alginsäure. Diese organischen Oberflächenbeschichtungen können allein, in Kombination und/oder in Kombination mit anorganischen Beschichtungsmaterialien vorkommen.

Erfindungsgemäß vorteilhafte Ausführungsformen der vorliegenden Erfindung sind **dadurch gekennzeichnet, dass** die erfindungsgemäße Zubereitung ein oder mehrere Verbindungen gewählt aus der Gruppe der Alkohole, Diole, Glycole, Glycerin, Parabene enthält.

Es ist dabei erfindungsgemäß vorteilhaft, wenn die Gesamtmenge dieser Verbindungen von 0,1 bis 80 Gew.-% bezogen auf das Gesamtgewicht der Zubereitung, beträgt.

Die erfindungsgemäßen Zubereitungen können ferner vorteilhaft auch Selbstbräunungssubstanzen enthalten, wie beispielsweise Dihydroxyaceton und/oder Melaninderivate in Konzentrationen von 1 Gew.-% bis zu 10 Gew.-%, bezogen auf das Gesamtgewicht der Zubereitung.

Ferner vorteilhaft können die erfindungsgemäßen Zubereitungen auch Repellentien zum Schutz vor Mücken, Zecken und Spinnen und dergleichen enthalten. Vorteilhaft sind z. B. N,N-Diethyl-3-methylbenzamid (Handelsbezeichnung: Meta-delphene, "DEET"), Dimethylphtalat (Handelsbezeichnung: Palatinol M, DMP), 1-Piperidincarbonsäure-2-(2-hydroxyethyl)-1-methylpropylester sowie insbesondere 3-(N-n-Butyl-N-acetyl-amino)-propionsäureethylester (unter dem Handelsnamen Insekt Repellent® 3535 bei der Fa. Merck erhältlich). Die Repellentien können sowohl einzeln als auch in Kombination eingesetzt werden.

Als Feuchthaltemittel (Moisturizer) werden Stoffe oder Stoffgemische bezeichnet, welche kosmetischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.

Vorteilhafte Feuchthaltemittel (Moisturizer) im Sinne der vorliegenden Erfindung sind beispielsweise Glycerin, Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist. Moisturizer können vorteilhaft auch als Antifaltenwirkstoffe zum Schutz vor Hautveränderungen, wie sie z. B. bei der Hautalterung auftreten, verwendet werden.

Es ist vorteilhaft im Sinne der vorliegenden Erfindung, wenn die erfindungsgemäße Zubereitung ein oder mehrere Feuchthaltemittel in einer Gesamtkonzentration von 0,1 bis 20 Gewichts-% und bevorzugt in einer Gesamtkonzentration von 0,5 bis 10 Gewichts-%, jeweils bezogen auf das Gesamtgewicht der Zubereitung, enthält.

Die erfindungsgemäßen kosmetischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben (wie z. B. Bornitrid etc.) und/oder Aerosile^{®} (CAS-Nr. 7631-86-9) und /oder Talkum.

Die Wasserphase der erfindungsgemäßen Zubereitungen kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, 2-Methylpropan-1,3-diol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, Polymere, Schaumstabilisatoren, Elektrolyte, Selbstbräuner sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination. Weitere erfindungsgemäß vorteilhafte Verdicker sind solche mit der INCI-Bezeichnung Acrylates/C10-30 Alkyl Acrylate Crosspolymer (z. B. Pemulen TR 1, Pemulen TR 2, Carbopol 1328 von der Fa. NOVEON) sowie Aristoflex AVC (INCI: Ammonium Acryloyldimethyltaurate/VP Copolymer).

Erfindungsgemäß vorteilhaft enthält die erfindungsgemäße Zubereitung Filmbildner. Filmbildner im Sinne der vorliegenden Erfindung sind Stoffe unterschiedlicher Zusammensetzung, die durch die folgende Eigenschaft charakterisiert sind: Löst man einen Filmbildner in Wasser oder anderen geeigneten Lösungsmitteln und trägt die Lösung dann auf die Haut auf, so bildet er nach dem Verdunsten des Lösemittels einen Film aus, der im wesentlichen dazu dient, die Lichtfilter auf der Haut zu fixieren und so die Wasserfestigkeit des Produktes zu steigern.

Es ist insbesondere von Vorteil, die Filmbildner aus der Gruppe der Polymere auf Basis von Polyvinylpyrrolidon (PVP) 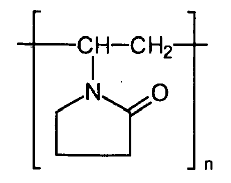 zu wählen. Besonders bevorzugt sind Copolymere des Polyvinylpyrrolidons, beispielsweise das PVP Hexadecen Copolymer und das PVP Eicosen Copolymer, welche unter den Handelsbezeichnungen Antaron V216 und Antaron V220 bei der GAF Chemicals Cooperation erhältlich sind.

Ebenfalls vorteilhaft sind weitere polymere Filmbildner, wie beispielsweise Natriumpolystryrensulfonat, welches unter der Handelsbezeichnung Flexan 130 bei der National Starch and Chemical Corp. erhältlich ist, und/oder Polyisobuten, erhältlich bei Rewo unter der Handelsbezeichnung Rewopal PIB1000. Weitere geeignete Polymere sind z.B. Polyacrylamide (Seppigel 305), Polyvinylalkohole, PVP, PVP / VA Copolymere, Polyglycole, Acrylat/Octylacralymid Copolymer (Dermacryl 79). Ebenfalls vorteilhaft ist die Verwendung von Hydriertem Rizinusöl Dimerdilinoleat (CAS 646054-62-8, INCI Hydrogenated Castor Oil Dimer Dilinoleate), das bei der Firma Kokyu Alcohol Kogyo unter dem Namen Risocast DA-H erworben werden kann oder aber auch PPG-3 Benzylethermyristat (CAS 403517-45-3), das unter dem Handelsnamen Crodamol STS bei der Firma Croda Chemicals erworben werden kann.

Die Ölphase der erfindungsgemäßen Zubereitung wird vorteilhaft gewählt aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Jojobaöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr.

Erfindungsgemäß vorteilhaft sind ferner z. B. natürliche Wachse tierischen und pflanzlichen Ursprungs, wie beispielsweise Bienenwachs und andere Insektenwachse sowie Beerenwachs, Sheabutter und/oder Lanolin (Wollwachs).

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Phenethylbenzoat, 2-Phenylethylbenzoat, Isopropyl Lauroyl Sarkosinat, Phenyl Trimethicon, Cyclomethicon, Dibutyladipat, Octylpalmitat, Octylcocoat, Octylisostearat, Octyldodeceylmyristat, Octyldodekanol, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl.

Ferner kann die Ölphase vorteilhaft gewählt werden aus der Gruppe der Dialkylether und Dialkylcarbonate, vorteilhaft sind z. B. Dicaprylylether (*Cetiol OE*) und/oder Dicaprylylcarbonat, beispielsweise das unter der Handelsbezeichnung *Cetiol CC* bei der Fa. Cognis erhältliche.

Es ist ferner bevorzugt, das oder die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, C₁₂₋₁₃-Alkyllactat, Di-C₁₂₋₁₃-Alkyltartrat, Triisostearin, Dipentaerythrityl Hexacaprylat/Hexacaprat, Propylenglykolmonoisostearat, Tricaprylin, Dimethylisosorbid. Es ist insbesondere vorteilhaft, wenn die Ölphase der erfindungsgemäßen Formulierungen einen Gehalt an C₁₂₋₁₅-Alkylbenzoat aufweist oder vollständig aus diesem besteht.

Vorteilhafte Ölkomponenten sind ferner z. B. Butyloctylsalicylat (beispielsweise das unter der Handelsbezeichnung *Hallbrite BHB* bei der Fa. CP Hall erhältliche), Tridecylsalicylat (welches unter der Handelsbezeichnung Cosmacol ESI bei der Fa. Sasol erhältlich ist), C12-C15 Alkylsalicylat (unter der Handelsbezeichnung Dermol NS bei der Fa. Alzo erhältlich), Hexadecylbenzoat und Butyloctylbenzoat und Gemische davon (*Hallstar AB*) und/oder Diethylhexylnaphthalat *(Hallbrite TQ oder Corapan TQ von Symrise).*

Auch beliebige Abmischungen solcher Öl- und Wachskomponenten sind vorteilhaft im Sinne der vorliegenden Erfindung einzusetzen.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene, C13-16 Isoparaffin und Isohexadecan. Unter den Polyolefinen sind Polydecene die bevorzugten Substanzen.

Die erfindungsgemäßen Zubereitungen können ferner vorteilhaft eine oder mehrere Substanzen aus der folgenden Gruppe der Siloxanelastomere enthalten, beispielsweise um die Wasserfestigkeit und/oder den Lichtschutzfaktor der Produkte zu steigern:
(a) Siloxanelastomere, welche die Einheiten R₂SiO und RSiO_{1,5} und/oder R₃SiO_{0,5} und/oder SiO₂ enthalten,
   wobei die einzelnen Reste R jeweils unabhängig voneinander Wasserstoff, C₁₋₂₄-Alkyl (wie beispielsweise Methyl, Ethyl, Propyl) oder Aryl (wie beispielsweise Phenyl oder Tolyl), Alkenyl (wie beispielsweise Vinyl) bedeuten und das Gewichtsverhältnis der Einheiten R₂SiO zu RSiO_{1,5} aus dem Bereich von 1 : 1 bis 30 : 1 gewählt wird;
(b) Siloxanelastomere, welche in Silikonöl unlöslich und quellfähig sind, die durch die Additionsreaktion eines Organopolysiloxans (1), das siliciumbebundenen Wasserstoff enthält, mit einem Organopolysiloxan (2), das ungesättigte aliphatische Gruppen enthält, erhältlich sind,
   wobei die verwendeten Mengenateile so gewählt werden, dass die Menge des Wasserstoffes des Organopolysiloxans (1) oder der ungesättigten aliphatischen Gruppen des Organopolysiloxans (2)
   - im Bereich von 1 bis 20 mol-% liegt, wenn das Organopolysiloxan nicht zyklisch ist und
   - im Bereich von 1 bis 50 mol-% liegt, wenn das Organopolysiloxan zyklisch ist.

Vorteilhaft im Sinne der vorliegenden Erfindung liegen das oder die Siloxanelastomere in Form sphärischer Puder oder in Form von Gelen vor.

Erfindungsgemäß vorteilhafte in Form sphärischer Puder vorliegende Siloxanelastomere sind die mit der INCI-Bezeichnung Dimethicone / Vinyl Dimethicone Crosspolymer, beispielsweise das von DOW CORNING unter der Handelsbezeichnungen DOW CORNING 9506 Powder erhältliche.

Besonders bevorzugt ist es, wenn das Siloxanelastomer in Kombination mit Ölen aus Kohlenwasserstoffen tierischer und/oder pflanzlicher Herkunft, synthetischen Ölen, synthetischen Estern, synthetischen Ethern oder deren Gemischen verwendet wird.

Besonders vorteilhafte Zubereitungen werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien können alle für kosmetische Anwendungen geeigneten oder gebräuchlichen Antioxidantien verwendet werden.

Besonders vorteilhaft im Sinne der vorliegenden Erfindung können wasserlösliche Antioxidantien eingesetzt werden, wie beispielsweise Vitamine, z. B. Ascorbinsäure und deren Derivate.

Bevorzugte Antioxidantien sind ferner Vitamin E und dessen Derivate sowie Vitamin A und dessen Derivate.

Die Menge der Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern Vitamin A bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Es ist insbesondere vorteilhaft, wenn die kosmetischen Zubereitungen gemäß der vorliegenden Erfindung kosmetische Wirkstoffe enthalten, wobei bevorzugte Wirkstoffe Antioxidantien sind, welche die Haut vor oxidativer Beanspruchung schützen können.

Weitere vorteilhafte Wirkstoffe im Sinne der vorliegenden Erfindung sind natürliche Wirkstoffe und/oder deren Derivate, wie z. B. alpha-Liponsäure, Folsäure, Phytoen, D-Biotin, Coenzym Q10, alpha-Glucosylrutin, Carnitin, Carnosin, natürliche und/oder synthetische Isoflavonoide, Kreatin, Kreatinin, Taurin und/oder β-Alanin sowie 8-Hexadecen-1,16-dicarbonsäure (Dioic αcid, CAS-Nummer 20701-68-2; vorläufige INCI-Bezeichnung Octadecendioic acid) und/oder Licochalcon A.

Erfindungsgemäße Rezepturen, welche z. B. bekannte Antifaltenwirkstoffe wie Flavonglycoside (insbesondere α-Glycosylrutin), Coenzym Q10, Vitamin E und/oder Derivate und dergleichen enthalten, eignen sich insbesondere vorteilhaft zum Schutz vor ästhetisch unattraktiven Hautveränderungen, wie sie z. B. bei der Hautalterung auftreten (wie beispielsweise Trockenheit, Rauhigkeit und Ausbildung von Trockenheitsfältchen, Juckreiz, verminderte Rückfettung (z. B. nach dem Waschen), sichtbare Gefäßerweiterungen (Teleangiektasien, Cuperosis), Schlaffheit und Ausbildung von Falten und Fältchen, lokale Hyper-, Hypo- und Fehlpigmentierungen (z. B. Altersflecken), vergrößerte Anfälligkeit gegenüber mechanischem Stress (z. B. Rissigkeit) und dergleichen) und ermüdete Haut. Weiterhin vorteilhaft eignen sie sich gegen das Erscheinungsbild der trockenen bzw. rauhen Haut.

Die kosmetischen Zubereitungen gemäß der Erfindung können kosmetische Hilfsstoffe enthalten, wie sie üblicherweise in solchen Zubereitungen verwendet werden, z. B. Konservierungsmittel, Konservierungshelfer, Komplexbildner, Bakterizide, Parfüme, Substanzen zum Verhindern oder Steigern des Schäumens, Farbstoffe, Pigmente, die eine färbende Wirkung haben, Verdickungsmittel, anfeuchtende und/oder feuchhaltende Substanzen, Füllstoffe, die das Hautgefühl verbessern, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Vorteilhaft im Sinne der vorliegenden Erfindung sind Zubereitungen zur Pflege der Haut: sie können dem kosmetischen Lichtschutz, ferner als Schminkprodukt in der dekorativen Kosmetik dienen.

Entsprechend ihrem Aufbau können kosmetische Zusammensetzungen im Sinne der vorliegenden Erfindung, beispielsweise verwendet werden als Hautschutzcrème, Tages- oder Nachtcrème usw. Es ist gegebenenfalls möglich und vorteilhaft, die erfindungsgemäßen Zusammensetzungen als Grundlage für pharmazeutische Formulierungen zu verwenden.

Es ist auch vorteilhaft im Sinne der vorliegenden Erfindung, kosmetische Zubereitungen zu erstellen, deren hauptsächlicher Zweck nicht der Schutz vor Sonnenlicht ist, die aber dennoch einen Gehalt an UV-Schutzsubstanzen enthalten. So werden z. B. in Tagescrèmes oder Makeup-Produkten gewöhnlich UV-A- bzw. UV-B-Filtersubstanzen eingearbeitet. Auch stellen UV-Schutzsubstanzen, ebenso wie Antioxidantien und, gewünschtenfalls, Konservierungsstoffe, einen wirksamen Schutz der Zubereitungen selbst gegen Verderb dar. Günstig sind ferner kosmetische Zubereitungen, die in der Form eines Sonnenschutzmittels vorliegen. Erfindungsgemäß ist insbesondere die Verwendung der erfindungsgemäßen Zubereitung zum Schutz vor Hautalterung (insbesondere zum Schutz vor UV-bedingter Hautalterung) sowie als Sonnenschutzmittel.

Erfindungsgemäß vorteilhaft weist die erfindungsgemäße Zubereitung einen pH-Wert von 5 bis 8 auf. Dieser kann durch die herkömmlichen Säuren, Basen und Puffersysteme eingestellt werden.

Zur Anwendung werden die erfindungsgemäßen kosmetischen Zubereitungen in der für Kosmetika üblichen Weise auf die Haut und/oder die Haare in ausreichender Menge aufgebracht.

Erfindungsgemäß ist die Verwendung der Verbindung 1 in kosmetischen Zubereitungen (insbesondere Sonnenschutzmitteln) zur Erhöhung der Auswaschbarkeit der Zubereitung aus Textilien.

Erfindungsgemäß ist die Verwendung der Verbindung 1 in kosmetischen Sprays (insbesondere Sonnenschutzmitteln).

Erfindungsgemäß ist die Verwendung der Verbindung 1 in kosmetischen Zubereitungen (insbesondere Sonnenschutzmitteln), die für die Tränkung kosmetischer Tücher vorgesehen sind.

### Vergleichsversuche

### Vergleichsversuch 1

Die Verfärbung von Textilien (z.B. Baumwolle) lässt sich über die Bestimmung von LAB-Werten mittels Spectro PEN erfassen. Das Verfahren wird im folgenden stichpunktartig erläutert:
• Messinstrument: **Spectro Pen** (Dr. Lange, Düsseldorf, D)
• Richtlinie: Working Instruction 4 STU 309
• Kenngrößen: CIE L*a*b*-System: L*-Wert als Maß für die Helligkeit, a*-Wert als Maß für die Rötung sowie b*-Wert als Maß für die Gelb- bzw. Blaufärbung
• 1 g Produktes wird auf das Textilstück (Areal: 2 x 2 cm) aufgebracht, anschließende Trocknung bei Raumtemperatur über Nacht
• Standardisierter Waschvorgang (Eumenia Single nova 1000, 40 g Persil Megaperls, Koch /Buntwäsche 60°C)
• Messungen je Areal: 6; Auswertung der Originalwerte
• Interpretation: Erhöhung der b*-Werte als Zeichen einer gelben Textilverfärbung

| | Probe 1 | Probe 2 | Probe 3 |
|---|---|---|---|
| Alkohol | 4 | 4 | 4 |
| Glyceryl Stearate SE | 1 | 1 | 1 |
| Cetearyl Alkohol + PEG-40 Rizinusöl + Natrium Cetearyl Sulfat | 2,5 | 2,5 | 2,5 |
| PVP Hexadecene Copolymer | 0,5 | 0,5 | 0,5 |
| TEDTA | 1 | 1 | 1 |
| Konservierung | q.s | q.s | q.s |
| Glycerin | 7,5 | 7,5 | 7,5 |
| Dibutyladipat | 3 | 3 | 3 |
| C12-15 Alkyl Benzoat | 5,6 | 5,6 | 5,6 |
| Parfum | q.s | q.s | q.s |
| Phenethylbenzoat | 5,4 | 5,4 | 5,4 |
| Butyl Methoxydibenzoylmethan | 0 | 2 | 0 |
| Verbindung 1 | 0 | 0 | 2 |
| Xanthan Gummi | 0,4 | 0,4 | 0,4 |
| Cetyl Alkohol | 1,5 | 1,5 | 1,5 |
| Vitamin E Acetat | 0,5 | 0,5 | 0,5 |
| Wasser | add 100 | add 100 | add 100 |

Folgende b*-Werte wurden ermittelt

| Probe | b*-Wert | visuelle Beurteilung |
|---|---|---|
| 1 | 2,2 | Sehr gut |
| 2 | 8,3 | befriedigend |
| 3 | 6,3 | gut |

Der Vergleichsversuch zeigt, dass eine Zubereitung mit der Verbindung 1 bei gleicher Filtermenge die Textilien deutlich weniger gelb verfärbt als Zubereitungen mit dem bekannten UV-A-Filter Butyl Methoxydibenzoylmethan.

### Vergleichsversuch 2

Der folgende Versuch verdeutlicht die Erhöhung der UV-A-Balance

| | Probe 1 | Probe 2 |
|---|---|---|
| Octyldodecanol | 5,5 | 5,5 |
| Glycerin | 7,5 | 0 |
| Vitamin E Acetate | 0,5 | 0,5 |
| EDTA | 1 | 1 |
| Ethylhexyl Methoxycinnamat | 2 | 2 |
| Konservierung | q.s. | q.s. |
| Cyclomethicon | 2 | 2 |
| Hydrogenated Coco-Glycerides | 1 | 1 |
| C12-15 Alkylbenzoat | 7 | 7 |
| Butyl Methoxydibenzoylmethan | 3 | 0 |
| Verbindung1 | 0 | 3 |
| PVP Hexadecen Copolymer | 0,5 | 0,5 |
| Glycerin | 0 | 6,55 |
| Xanthan Gummi | 0,4 | 0,4 |
| Acrylat/C10-30 Alkyl Acrylat Crosspolymer | 0,1 | 0,1 |
| Stearyl Alkohol | 2 | 2 |
| Dicaprylyl Ether | 5,5 | 5,5 |
| Octocrylen | 1 | 1 |
| Bis-Ethylhexyloxyphenol Methoxyphenyl Triazin | 1,7 | 1,7 |
| Glyceryl Stearat Citrat | 2 | 2 |
| Parfum | q.s. | q.s. |
| Wasser | add 100 | add 1005 |

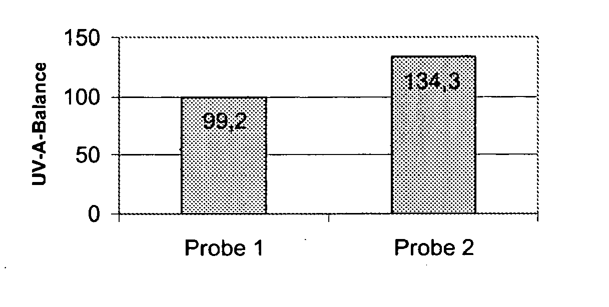

**Beispiele:**

**Beispiele für Gele**

| | 1 | 2 | 3 | 4 | 5 | 6 |
|---|---|---|---|---|---|---|
| Acrylates/Octylacrylamide Copolymer | 1,0 | 1,0 | 1,0 | 1,0 | 1,0 | 1,0 |
| Alohol Denat. | 50,0 | 62,0 | 59,2 | 70,0 | 70,0 | 69,0 |
| Butyl Methoxydibenzoylmethan | 4,5 | 4,5 | | | 2,5 | 4,5 |
| Butylene Glycol Dicaprylat/Dicaprat | | | 7,5 | | | 2 |
| C12-15 Alkyl Benzoate | 5,0 | 8,5 | 5,0 | 2 | 7,5 | |
| Phenylethylbenzoat | 3,0 | | | 2,5 | | |
| Cocoglycerid | | | | | | 2 |
| Tridecylsalicylat | 2 | 1,5 | | | 3 | 1 |
| Diethylamino Hydroxybenzoyl Hexyl Benzoate | | | 4,5 | 3,5 | | |
| Ethylhexyl Methoxycinnamat | 5 | 9,5 | | 6,5 | 6,5 | 9,5 |
| Ethylhexyl Salicylat | 4,5 | 4,5 | 4,5 | 4,5 | | |
| Homosalat | | | | | | 4,5 |
| Hydroxypropylcellulose | 2 | 0,8 | 1 | 0,8 | 0,5 | 0,8 |
| Octocrylen | 9,5 | 4,8 | 9,5 | 4,3 | 3,8 | |
| Ethylhexyltriazin | | | | | 2 | |
| Benzophenon-3 | 3 | | | | | |
| Drometrizol Trisiloxan | | | | 0,5 | 1,0 | |
| Verbindung 1 | 0,5 | 1,5 | 2,5 | 5 | 3,5 | 0,1 |
| Bis-Ethylhexyloxyphenol Methoxy-phenyltriazin | | | | | | 1 |
| Vitamin E Acetat | | | | 0,5 | | 0,2 |
| Glycerin | 5 | | 3 | | | |
| Parfüm, Farbstoffe | q.s. | q.s. | q.s. | q.s. | q.s. | q.s. |
| Wasser | ad 100 | ad 100 | ad 100 | ad 100 | ad 100 | ad 100 |

**Beispiele für Sprays**

| | 1 | 2 | 3 | 4 | 5 | 6 |
|---|---|---|---|---|---|---|
| Acrylat/Octylacrylamid Copolymer | 1 | | | | 1 | 1 |
| VP/VA Copolymer | | 1,0 | | 0,5 | | |
| Alkohol Denat. | 42 | 57 | 60 | 53 | 35,5 | 43,5 |
| Bis-Ethylhexyloxyphenol Methoxyphenyl Triazin | | | | | 1,0 | |
| Uvasorb® K2A | 0,5 | | 1,0 | | | 3,0 |
| Butyl Methoxydibenzoylmethan | 4,5 | 2 | | 3 | 4,5 | |
| Cyclomethicon | 4,9 | 2 | 5 | 0,5 | 8,0 | 8 |
| Diethylamino Hydroxybenzoyl Hexyl Benzoat | | 2 | 3 | | | 4,5 |
| Ethylhexyl Methoxycinnamat | 9,5 | | | 7 | 6,5 | 9,5 |
| Ethylhexyl Salicylat | 4,5 | 3,5 | 2 | 4 | 4,5 | 2,5 |
| Drometrizol Trisiloxan | | 0,5 | | | | 2,0 |
| Tridecylsalicylat | 0,5 | 2,5 | 4 | 7 | 10 | 3 |
| Homosalat | 9,5 | 5 | 3 | | 9,5 | 6,5 |
| Octocrylen | 9,5 | | 8 | | 7,5 | 9,5 |
| Verbindung 1 | 0,5 | 2,5 | 6,8 | 1 | 2 | 4 |
| Merocyanin | | | 2 | | | |
| Butylen Glycol Dicaprylat/Dicaprat | | 9 | | | | |
| C12-15 Alkyl Benzoat | | 2 | | 2 | | |
| Phenylbenzoat | | | 7 | | 4 | |
| Diethylhexylnaphthalat | | 6 | | 3 | | |
| Isopropyl Lauroyl Sarkosinat | | | 2 1 3 | | | |
| Phenyl Trimethicone | 2 5 | | | | 1 2 | |
| Octyldodekanol | | | | 8 | | |
| Glycerin | 5 | 4 | 5 | 8 | 5 | 5 |
| Vitamin E Acetat | 0,1 | | 0,5 | | | |
| Parfüm, Farbstoffe | q.s. | q.s. | q.s. | q.s. | q.s. | q.s. |
| Wasser | ad 100 | ad 100 | ad 100 | ad 100 | ad 100 | ad 100 |

**O/W-Emulsionen**

| | 1 | 2 | 3 | 4 | 5 | 6 |
|---|---|---|---|---|---|---|
| Glyceryl Stearat Citrat | 2 | 2 | 3 | | | |
| Glyceryl Stearat SE | | | | 1 | 1 | 1,5 |
| Cetearyl Alkohol + PEG-40 Rizinusöl+ Natrium Cetearyl Sulfat | | | | 2,5 | 2,5 | 3 |
| Cetearylalkohol | | | 1 | 1 | | |
| Stearylalkohol | 0,5 | | | | | 2 |
| Myristylmyristat | 1,0 | 1 | | | 3 | |
| Acrylates/C₁₀₋₃₀ Alkyl Acrylat Cross-polymer | 0,1 | 0,2 | | | 0,1 | |
| Carbomer | | 0,2 | 0,3 | 0,2 | | |
| Xanthan Gum | 0,4 | | 0,2 | 0,2 | 0,3 | 0,4 |
| C₁₂₋₁₅ Alkyl Benzoat | | 3 | | | 5 | |
| 2-Phenylethylbenzoat | 5 | 2 | | | | |
| Butylenglycol Dicaprylat/Dicaprat | 5 | | | | 3 | 3 |
| Tridecylsalicylat | 1,5 | 2,5 | 0,25 | 5,9 | 7 | 15 |
| Dicaprylcaprat | 2 | 2 | | | 2 | 2 |
| Cyclomethicon | | | 5 | 10 | | |
| Dimethicon | | | | 5 | | |
| PVP Hexadecen Copolymer | | 0,5 | | | | 1 |
| Propylenglykol | | | 1 | | 5 3 | |
| Glycerin | 7,5 | 5 | 7 | 10 | 13 | 3 |
| Alkohol denat. | 2 3 | | | 7 | | |
| Titandioxid | | 3 2 | | | | |
| Merocyanin | 2 | | 2 | | 3 | 3 |
| Phenylen-1,4-bis-(2-benzimidazyl)-3,3'-5,5'-tetrasulfonsäuresalz | 3 | 2 | | | | |
| Ethylhexyltriazin | 2,5 | 2 | | 1 | | |
| Phenylbenzimidazol Sulfonsäure | | | 2 | | | 1 |
| 4-Methoxyzimtsäure(2-ethylhexyl)ester | 9,5 | 5 | | 2 | | |
| Butyl Methoxydibenzoylmethan | | 1 | | | 3 | |
| Ethylhexylsalicylat | 2 | | 0,5 | 4 | 9,5 | |
| Octocrylen | | | | | 5 | 7 |
| Bis-Ethylhexyloxyphenol Methoxyphenyltriazin | | 1 | | 1 | | 1 |
| Verbindung 1 | 2 | 3 | 4 | 1 | 0,25 | 5 |
| Vitamin E Acetat | 0,2 | 0,2 | 0,2 | 0,3 | 0,1 | 0,5 |
| Na₂H₂EDTA | 0,1 | 0,1 | 0,2 | 0,2 | 0,2 | 0,5 |
| Parfüm, Konservierungsmittel | q.s. | q.s. | q.s. | q.s. | q.s. | q.s. |
| Farbstoffe, usw. | q.s. | q.s. | q.s. | q.s. | q.s. | q.s. |
| Citronensäure, Natriumcitrat | q.s. | q.s. | q.s. | q.s. | q.s. | q.s. |
| Natriumhydroxid | q.s. | q.s. | q.s. | q.s. | q.s. | q.s. |
| Wasser | ad 100,0 | ad 100,0 | ad 100,0 | ad 100,0 | ad 100,0 | ad 100,0 |
| | | | | | | |
| | 7 | 8 | 9 | 10 | 11 | 12 |
| Glycerylstearat | 2,5 | 2 | 1,2 | 1 | | |
| PEG-40 Stearat | 1 | | | | | |
| PEG-100 Stearat | | 2,5 | | | | |
| Ceteareth-20 | | | | | 1,0 | |
| Glyceryl Stearat Citrat | | | | | | 0,5 |
| Stearinsäure | | | 2,5 | 3 | | |
| Cetearylalkohol | 4,0 | | | 2 | | |
| Stearylalkohol | | 2 | 1 | | | |
| Cetylalkohol | | | 1 | 1 | | |
| Acrylates/C₁₀₋₃₀ Alkyl Acrylat Cross-polymer | | | | 0,2 | 0,2 | 0,4 |
| Carbomer | 0,1 | | 0,2 | | | |
| Xanthan Gum | | 0,3 | | | | |
| C₁₂₋₁₅ Alkyl Benzoat | 5 | | | 2 | 5 | 5 |
| Vaseline | 5 | | 3 | | | |
| Butylenglycol Dicaprylat/Dicaprat | | 4 | 2 | | 9 | |
| Hydrogeniertes Polydecen | | | 3 | | 2 | |
| Caprylic/Capric Triglycerid | 1 | 3 | | 5 | | 5 |
| Cyclomethicon | | 5 | 2 | | | 10 |
| Metylpropandiol | 2 | | | | 3 | |
| Isopropyl Lauroyl Sarkosinat | | | | | | |
| Glycerin | 7,5 | 10 | 4 | 5 | 5 | |
| Ethanol | 1 | 3 | 0,5 | 10 | 4 | 8 |
| Butylen Glykol | | | 3 | | | |
| Titandioxid | 1 | | 0,5 | 2 | | |
| 2,4,6-Tribiphenyl-4-yl-1,3,5-triazin | | | 0,5 | 4 | | 6 |
| Dimethicodiethylbenzalmalonat | | | | 2 | | |
| Ethylhexylmethoxycinnamat | 4 | | | | 2 | |
| Phenylbenzimidazol Sulfonsäure | 1,5 | | | 2 | | 2 |
| Butyl Methoxydibenzoylmethan | 2,5 | | | 2 | | 3 |
| Isotridecylsalicylat | | | 1 | 3 | 5 | 2 |
| Verbindung 1 | 1 | 2 | 4 | 0,75 | 5 | 2,5 |
| Octocrylen | | 5 | | | | 2 |
| Octylsalicylat | 3 | | | | 5 | |
| Homosalat | | | 4 | | 2 | |
| Drometrizol Trisiloxan | 0,5 | | | 1 | | 2 |
| Terephthaliden Dicampher Sulfonsäure | 0,75 | | | 0,5 | | 0,25 |
| Tapiokastärke | 1 | | 2,5 | | | 0,5 |
| Natrium-Stärke Octenylsuccinat | | | | 1 | | |
| Na₂H₂EDTA | 0,1 | | | | | 0,5 |
| Parfüm, Konservierungsmittel | q.s. | q.s. | q.s. | q.s. | q.s. | q.s. |
| Natriumhydroxid | q.s. | q.s. | q.s. | q.s. | q.s. | q.s. |
| Wasser | ad 100,0 | ad 100,0 | ad 100,0 | ad 100,0 | ad 100,0 | ad 100,0 |

**W/O-Emulsionen**

| | 1 | 2 | 3 | 4 | 5 | 6 |
|---|---|---|---|---|---|---|
| Polyglyceryl-2 Dipolyhydroxystearat | 3 | 5 3 | | | | |
| PEG-30 Dipolyhydroxystearat | | | 2 | 3 | 4 | 5 |
| Natrium-Stärke Octenylsuccinat | 0,5 | 0,4 | 0,6 | 0,3 | 0,5 | 1 |
| Glycin | 0,3 | 0,3 | 0,5 | 0,4 | | |
| Alkohol | 2 | 5 | 2 | 0,5 | 8 | 1 |
| Magnesiumsulfat | 0,2 | 0,3 | 0,3 | 0,4 | 0,5 | 0,2 |
| C₁₂₋₁₅ Alkyl Benzoat | 5 3 | | | | 5 | |
| Triheptanoin | | 2 | | | | |
| Butylenglycol Dicaprylat/Dicaprat | 5 | | | | 3 | 3 |
| Dicaprylyl Ether | | | | | 2 | |
| Mineralöl | | 4 | | 6 | | 8 |
| Octyldodecanol | 2 | | | | | |
| Dicaprylcaprat | | 2 | | | 2 | 2 |
| Cyclomethicon | 5 | | 5 | 10 | | |
| Dimethicon | | | | 5 | | |
| Isohexadecan | | 1 | | | | |
| Butylenglycol | 5 | 8 | | | | 3 |
| Glycerin | 3 | 5 | 7 | 10 | 3 | 3 |
| Tridecylsalicylat | | 1 | 2 | | | 0,5 |
| Ethylhexylmethoxycinnamat | 5 | | 7 | | 5 | |
| Ethylhexyltriazin | 2 | 3 | 3 | | | 3 |
| Diethylhexylbutamidotriazin | | 1,5 | 1,5 | | | 1,5 |
| Butyl Methoxydibenzoylmethan | | 4 | | | | |
| 2-Phenylethylbenzoat | | | 2 | | 4 | |
| Isopropyl Lauroyl Sarkosinat | | | 1 | | 2 | |
| 2-(4'-Diethylamino-2'-hydoxybenzoyl)-benzoesäurehexylester | | | | 2 | | |
| Verbindung 1 | 1 | 2 | 4 | 0,75 | 5 | 2,5 |
| Titandioxid | 5 | 4 | 2 | | 3 | 4 |
| Bis-Ethylhexyloxyphenol Methoxyphenyltriazin | 1 | 2 | 2 | | 2 | 3 |
| Methylen Bis-Benzotriazolyl Tetramethylbutylphenol | 2 | | 3 | | 1 | |
| Merocyanin | 2 | | 2 | 5 | 1 | |
| Vitamin E Acetat | 0,2 | 0,2 | 0,2 | 0,3 | 0,1 | 0,5 |
| Na₂H₂EDTA | 0,1 | 0,1 | 0,2 | 0,2 | 0,2 | 0,5 |
| Parfüm, Konservierungsmittel | q.s. | q.s. | q.s. | q.s. | q.s. | q.s. |
| Wasser | ad 100,0 | 100,0 | ad 100,0 | ad 100,0 | ad 100,0 | ad 100,0 |

**Hydrodispersionen**

| | 1 | 2 | 3 | 4 | 5 | 6 |
|---|---|---|---|---|---|---|
| Glyceryl Stearat Citrat | | 0,40 | | | | |
| Sodium Carbomer | | | | | 0,30 | |
| Acrylates/C10-30 Alkyl Acrylate Cross-polymer | | | 0,30 | 0,40 | 0,10 | 0,10 |
| Ceteareth-20 | | | 1,00 | | | |
| Xanthan Gummi | 0,50 | | | 0,15 | | 0,50 |
| Dimethicon / Vinyl Dimethicon Cross-polymer | | | | 5,00 | | 3,00 |
| 2-(4'-Diethylamino-2'-hydoxybenzoyl)-benzoesäurehexylester | 0,25 | | | 0,50 | 2,00 | 1,50 |
| Butyl Methoxydibenzoylmethan | 2 | | 3,50 | | | |
| Bis-Ethylhexyloxyphenol Methoxypheny Triazin | | 2,00 | | 0,25 | | |
| Dinatrium Phenyl Dibenzimidazol Tetrasulfonat | 0,75 | | | | | 1,00 |
| Phenylbenzimidazol Sulfonsäure | | | 2,00 | | | |
| Ethylhexyl Methoxycinnamat | | | 7,00 | | 5,00 | 8,00 |
| Diethylhexyl Butamido Triazin | | | 2,00 | 2,00 | | |
| Ethylhexyl Triazin | 4,00 | | 3,00 | | 4,00 | |
| Verbindung 1 | 1 | 2 | 4 | 0,75 | 5 | 2,5 |
| Titandioxid | 0,50 | 2,00 | 1,00 | 2,00 | 3,00 | 1,00 |
| Merocyanin | | | | | | |
| Drometrizol Trisiloxan | | | | 1 | | 0,5 |
| Terephthaliden Dicampher Sulfonsäure | | | | 0,5 | | 0,75 |
| C₁₂₋₁₅ Alkyl Benzoat | 2,00 | | 2,50 | | | |
| Butylenglycol Dicaprylat/Dicaprat | 4,00 | | | | 6,00 | |
| Dicaprylyl Carbonat | | 3,00 | | | | |
| Dicaprylylether | | 2,00 | | | | |
| Cyclomethicon | | | | 7,50 | | |
| 2-Phenylethylbenzoat | | | 4 | | 2 | |
| Diethylhexylnaphthalat | 5 | | 6 | | | |
| Tridecylsalicylat | 2 | 3 | 1 | 5 | 3 | 0,5 |
| PVP Hexadecen Copolymer | 0,50 | | 0,50 | | 0,50 | 1,00 |
| Glycerin | 10,00 | 5,00 | 5,00 | | 5,00 | 15,00 |
| Butylenglycol | | 7,00 | | | | |
| Glycin Soja | | | | 1,00 | | |
| Vitamin E Acetat | 0,50 | 0,25 | 0,50 | 0,25 | 0,75 | 1,00 |
| α - Glycosylrutin | | | | | 0,25 | |
| Trinatrium EDTA | | 1,00 | 1,00 | 0,10 | 0,20 | |
| Ethanol | 3,00 | 10,00 | 4,00 | 3,50 | 0,5 | 1,00 |
| Konservierungsmittel | q.s. | q.s. | q.s. | q.s. | q.s. | q.s. |
| Parfüm, Farbstoffe, | q.s. | q.s. | q.s. | q.s. | q.s. | q.s. |
| Wasser | ad 100 | ad 100 | ad 100 | ad 100 | ad 100 | ad 100 |

**kosmetische Schäume**

| **Emulsion** | **A** | **B** | **C** |
|---|---|---|---|
| Stearinsäure | 2 | 2 | |
| Palmitinsäure | | | 1,5 |
| Cetylalkohol | 2,5 | 2 | |
| Stearylalkohol | | | 3 |
| PEG-100 Stearat | | | 3,5 |
| PEG-40 Stearat | | 2 | |
| PEG-20-Stearat | 3 | | |
| Sorbitanstearat | | 0,8 | |
| C₁₂₋₁₅ Alkyl Benzoat | 5 | | |
| C₁₂₋₁₃ Alkyl Tartrat | | | 7 |
| Butylenglycol Dicaprylat/Dicaprat | | 6 | |
| Dicaprylyl Ether | | | 2 |
| Cyclomethicon | | 2 | 3 |
| Butylenglycol | 1 | | |
| Isohexadecan | 2 | | |
| Methylpropandiol | | | |
| Propylenglykol | | | 5 |
| Glycerin | 5 | 7 | |
| 2,4-bis-[5-1 (dimethylpropyl)benzoxazol-2-yl-(4-phenyl)-imino]-6-(2-ethylhexyl)-imino-1,3,5-triazin | | | 2 |
| 2-(4'-Diethylamino-2'-hydoxybenzoyl)-benzoesäurehexylester | 2 | | |
| Dimethicodiethylbenzalmalonat | | 3 | |
| Homosalat | | 5 | |
| Phenylbenzimidazol Sulfonsäure | | 2 | 2 |
| Benzophenon-3 | 2 | | |
| Octylsalicylat | | 5 | |
| Octocrylen | 2 | | |
| Bis-Ethylhexyloxyphenol Methoxyphenyltriazin | | 3 | |
| 2,2'-Methylen-bis-(6-(2H-benzotriazol-2-yl)-4-(1,1,3,3-tetramethylbutyl)-phenol) | | | 8 |
| 2,4,6-Tris-(biphenyl)-1,3,5-triazin | 5 | | |
| Verbindung 1 | 3 | 6 | 10 |
| C8-C16 Alkylpolyglycosid | 1 | | |
| Vitamin E Acetat | 0,6 | 0,5 | 0,2 |
| Kreatin/Kreatinin | | | 0,5 |
| BHT | | | 0,1 |
| Na₂H₂EDTA | 0,50 | | |
| Parfüm, Konservierungsmittel | q.s. | q.s. | q.s. |
| Farbstoffe, usw. | q.s. | q.s. | q.s. |
| Natriumhydroxid | q.s. | | q.s. |
| Kaliumhydroxid | | q.s. | |
| Wasser | ad 100,0 | ad 100,0 | ad 100,0 |

## Patentansprüche

1. Kosmetische Zubereitung enthaltend die Verbindung: 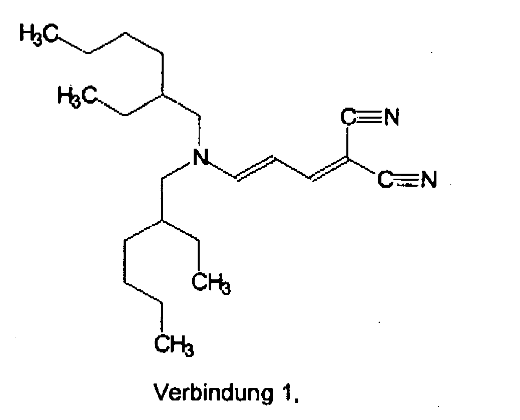 **dadurch gekennzeichnet, dass** sie ein oder mehrere Verbindungen gewählt aus der Gruppe der Alkohole, Diole, Glycole, Glycerin. Parabene enthält.

2. Kosmetische Zubereitung nach Anspruch 1, **dadurch gekennzeichnet, dass** sie die Verbindung 1 in einer Konzentration von 0,1 bis 10 Gewichts-%, bezogen auf das Gesamtgewicht der Zubereitung, enthält.

3. Kosmetische Zubereitung nach einem der Ansprüche 1 oder 2, **dadurch gekennzeichnet, dass** die Zubereitung in Form eines Gels, einer Emulsion, einer Dispersion oder eines Öls vorliegt.

4. Zubereitung nach einem der vorhergehenden Ansprüche, **dadurch gekennzeichnet, dass** die Zubereitung einen oder mehrere weitere UV-Filter enthält, gewählt aus der Gruppe der Verbindungen Phenylen-1,4-bis-(2-benzimidazyl)-3,3'-5,5'-tetrasulfonsäureselze, 2-Phenylbenzimidazol-5-sulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy)disiloxanyl]propyl]-phenol; 3-(4-Methylbenzyliden)campher; 3-Benzylidencampher; Ethylhexylsalicylat; Terephthalidendicamphersulfonsäure; 4-(Dimethylamino)-benzoesäure(2-ethylhexyl)ester; 4-(Dimethylamino)benzoesäure-amylester; 4-Methoxybenzalmalon-säuredi(2-ethylhexyl)-ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 2-(4'-Diethylamino-2'-hydoxybenzoyl)-benzoesäurehexylester, 4-(tert.-Butyl)-4'-methoxydibenzoylmethan; Homomenthylsalicylat; 2-Ethylhexyl-2-hydroxybenzoat; 2-Ethylhexyl-2-cyano-3,3-diphenylacrylat; Dimethicodiethylbenzalmalonat; 3-(4-(2,2-bis Ethoxycarbonylvinyl)-phenoxy)propenyl)-methoxysiloxan / Dimethylsiloxan - Copolymer; 2,4-Bis-{[4-(2-ethyl-hexyloxy)-2-hydroxyl-phenyl}-6-(4-mothoxyphenyl)-1,3,5-triazin (INCI :Bis-Ethylhexyloxyphenol Methoxyphenyl Triazin); Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoësäure-tris(2-ethylhexylester) (auch: 2,4,6-Tris-[anilinogp-carbo-2'-ethyl-1'-hexyloxy)]-1,3,5-triazin (INCI: Ethylhexyl Triazone); 2,4,6-Tribiphenyl-4-yl-1,3,5-triazin; Titiandioxid; Zinkoxid in einer Konzentration von 0,01 bis 40 Gewichts-% bezogen auf das Gesamtgewicht der Zubereitung.

5. Kosmetische Zubereitung nach einem der vorhergehenden Ansprüche, **dadurch gekennzeichnet, dass** die Zubereitung frei ist von p-Methylbenzylidencampher.

6. Kosmetische Zubereitung nach einem der vorhergehenden Ansprüche, **dadurch gekennzeichnet, dass** die Zubereitung in Form einer O/W-Emulsion vorliegt.

7. Kosmetische Zubereitung nach Anspruch 6, **dadurch gekennzeichnet, dass** die Zubereitung einen oder mehrere O/W-Emulgatoren gewählt aus der Gruppe der Verbindungen Glycerylstearatcitrat, Glycerylstearat (selbstemulgierend), Stearinsäure, Stearatsalze, Polyglyceryl-3-methylglycosedistearat, Ceteareth-20, PEG-40 Stearat , Natriumcetearylsulfat enthält.

8. Kosmetische Zubereitung nach einem der Ansprüche 1-5, **dadurch gekennzeichnet, dass** die Zubereitung in Form einer W/O-Emulsion vorliegt.

9. Kosmetische Zubereitung nach Anspruch 8, **dadurch gekennzeichnet, dass** die Zubereitung einen oder mehrere W/O-Emulgatoren gewählt aus der Gruppe der Verbindungen Polyglyceryl-2-dipolyhydroxystearat, PEG-30 Dipolyhydroxystearat, Cetyl Dimethicon Copolyol, Polyglyceryl-3 Diisostearat enthält.

## Claims

1. Cosmetic preparation comprising the compound: 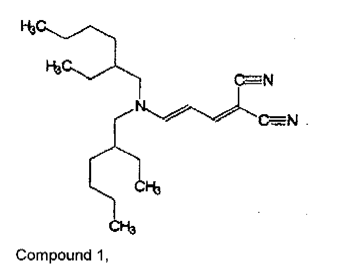 **characterized in that** it comprises one or more compounds selected from the group of alcohols, diols, glycols, glycerol, parabens.

2. Cosmetic preparation according to Claim 1, **characterized in that** it comprises the compound 1 in a concentration of from 0.1 to 10% by weight, based on the total weight of the preparation.

3. Cosmetic preparation according to either of Claims 1 and 2, **characterized in that** the preparation is present in the form of a gel, an emulsion, a dispersion or an oil.

4. Preparation according to one of the preceding claims, **characterized in that** the preparation comprises one or more further UV filters selected from the group of the compounds phenylene-1,4-bis(2-benzimidazyl)-3,3'-5,5'-tetrasulphonic acid salts; 2-phenylbenzimidazole-5-sulphonic acid salts; 1,4-di(2-oxo-10-sulpho-3-bornylidenemethyl)-benzene and salts thereof; 4-(2-oxo-3-bornylidenemethyl)benzenesulphonic acid salts; 2-methyl-5-(2-oxo-3-bornylidenemethyl)sulphonic acid salts; 2,2'-methylenobis(6-(2H-benzotriazol-2-yl)-4-(1,1,3,3-tetramethylbutyl)phenol); 2-(2H-benzotriazol-2-yl)-4-methyl-6-[2-methyl-3-[1,3,3,3-tetramethyl-1-[(trimethylsilyl)oxy]disiloxanyl]propyl]phenol; 3-(4-methylbenzylidene)camphor; 3-benzylidenecamphor; ethylhexyl salicylate; terephthalidenedicamphorsulphonic acid; 2-ethylhexyl 4-(dimethylamino)benzoate; amyl 4-(dimethylamino)benzoate, di(2-ethylhexyl) 4-methoxybenzalmalonate; 2-ethylhexyl 4-methoxycinnamate; isoamyl 4-methoxycinnamate; 2-hydroxy-4-methoxybenzophenone, 2-hydroxy-4-methoxy-4'-methylbenzophenone; 2,2'-dihydroxy-4-methoxybenzophenone; hexyl 2-(4'-diethylamino-2'-hydroxybenzoyl)benzoate, 4-(tert-butyl)-4'-methoxydibenzoylmethane; homomenthyl salicylate; 2-ethylhexyl 2-hydroxybenzoate; 2-ethylhexyl 2-cyano-3,3-diphenyl acrylate; dimethicodiethyl benzalmalonate; 3-(4-(2,2-bis-ethoxycarbonylvinyl)phenoxy)propenyl)methoxysiloxane/dimethylsiloxane copolymer; 2,4-bis{[4-(2-ethylhexyloxy)-2-hydroxy)phenyl}-6-(4-methoxyphenyl)-1,3,5-triazine (INCI: Bis-Ethylhexyloxyphenol Methoxyphenyl Triazine); dioctylbutylamidotriazone (INCI: Diethylhexyl-Butamidotriazone); 2,4-bis[5-1(dimethylpropyl)benzoxazol-2-yl-(4-phenyl)imino]-6-(2-ethylhexyl)imino-1,3,5-triazine with the CAS No. 288254-16-0; tris(2-ethylhexyl) 4,4',4"-(1,3,5-triazine-2,4,6-triyltriimino)trisbenzoate (also: 2,4,6-tris[anilino(p-carbo-2'-ethyl-1'-hexyloxy)]-1,3,5-triazine (INCI: Ethylhexyl Triazone); 2,4,6-tribiphenyl-4-yl-1,3,5-triazine; titanium dioxide; zinc oxide in a concentration of from 0.01 to 40% by weight, based on the total weight of the preparation.

5. Cosmetic preparation according to one of the preceding claims, **characterized in that** the preparation is free from p-methylbenzylidenecamphor.

6. Cosmetic preparation according to one of the preceding claims, **characterized in that** the preparation is present in the form of a O/W emulsion.

7. Cosmetic preparation according to Claim 6, **characterized in that** the preparation comprises one or more O/W emulsifiers selected from the group of the compounds glyceryl stearate citrate, glyceryl stearate (self-emulsifying), stearic acid, stearate salts, polyglyceryl-3 methylglucose distearate, Ceteareth-20, PEG-40 stearate, sodium cetearyl sulphate.

8. Cosmetic preparation according to one of Claims 1-5, **characterized in that** the preparation is present in the form of a W/O emulsion.

9. Cosmetic preparation according to Claim 8, **characterized in that** the preparation comprises one or more W/O emulsifiers selected from the group of the compounds polyglyceryl-2 dipolyhydroxystearate, PEG-30 dipolyhydroxystearate, cetyldimethicone copolyol, polyglyceryl-3 diisostearate.

## Revendications

1. Composition cosmétique contenant le composé : 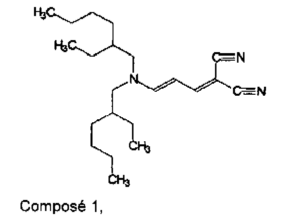 **caractérisée en ce qu'**elle contient un ou plusieurs composés choisis dans le groupe formé par les alcools, les diols, les glycols, le glycérol, les parabènes.

2. Composition cosmétique selon la revendication 1, **caractérisée en ce qu'**elle contient le composé 1 en une concentration de 0,1 à 10% en poids par rapport au poids total de la composition.

3. Composition cosmétique selon l'une quelconque des revendications 1 ou 2, **caractérisée en ce que** la composition se trouve sous forme d'un gel, d'une émulsion, d'une dispersion ou d'une huile.

4. Composition selon l'une quelconque des revendications précédentes, **caractérisée en ce que** la composition contient un ou plusieurs autres filtres des UV, choisis dans le groupe formé par les composés sels de l'acide phénylène-1,4-bis-(2-benzimidazyl)-3,3'-5,5'-tétrasulfonique ; sels de l'acide 2-phénylbenzimidazole-5-sulfonique ; 1,4-di(2-oxo-10-sulfo-3-bornylidèneméthyl)-benzène et ses sels ; sels de l'acide 4-(2-oxo-3-bornylidèneméthyl)benzènesulfonique ; sels de l'acide 2-méthyl-5-(2-oxo-3-bornylidèneméthyl)sulfonique ; 2,2'-méthylènebïs-(6-(2H-benzotriazol-2-yl)-4-(1,1,3,3-tétraméthylbutyl)-phénol) ; 2-(2H-benzotriazol-2-yl)-4-méthyl-6-[2-méthyl-3-[1,3,3,3-tétraméthyl-1-[(triméthylsilyl)oxy]disiloxanyl]propyl]-phénol ; 3-(4-méthylbenzylidène)camphre ; 3-benzylidènecamphre ; salicylate d'éthylhexyle ; acide téréphtalidènedicamphresulfonique ; ester 2-éthylhexylique de l'acide 4-(diméthylamino)-benzoïque ; ester amylique de l'acide 4-(diméthylamino)benzoïque ; ester di(2-éthylhexylique) de l'acide 4-méthoxybenzalmalonique ; ester 2-éthylhexylique de l'acide 4-méthoxycinnamique ; ester iso-amylique de l'acide 4-méthoxycinnamique ; 2-hydroxy-4-méthoxybenzophénone ; 2-hydroxy-4-méthoxy-4'-méthylbenzophénone ; 2,2'-dihydroxy-4-méthoxybenzophénone ; ester hexylique de l'acide 2-(4'-diéthylamino-2'-hydroxybenzoyl)-benzoïque, 4-(tert-butyl)-4'-méthoxydibenzoylméthane ; salicylate d'homomenthyle ; 2-hydroxybenzoate de 2-éthylhexyle : 2-cyano-3,3-diphénylacrylate de 2-éthylhexyle ; diméthicodiéthylbenzalmalonate ; copolymère de 3-(4-(2,2-bis-(éthoxycarbonylvinyl)-phénoxy)propényl)-méthoxysiloxane/diméthylsiloxane ; 2,4-bis-{[4-(2-éthylhexyloxy)-2-hydroxy]-phényl)-6-(4-méthoxyphényl)-1,3,5-triazine (INCI : Bis-Ethylhexyloxyphenol Methoxyphenyl Triazin) ; dioctylbutylamidotriazone (INCI : Diethylhexyl-Butamidotriazone) ; 2,4-bis-[5-1 (diméthylpropyl)benzoxazol-2-yl-(4-phényl)-imino]-6-(2-éthylhexyl)-imino-1,3,5-triazine (n° CAS 288254-16-0) ; ester tris(2-éthylhexylique) de l'acide 4,4',4"-(1,3,5-triazine-2,4,6-triyltriimino)-trisbenzoïque (également : 2,4,6-tris-[anilino-(p-carbo-2'-éthyl-1'-hexyloxy)]-1,3,5-triazine (INCI : Ethylhexyl Triazone) ; 2,4,6-tribiphényl-4-yl-1,3,5-triazine ; dioxyde de titane ; oxyde de zinc en une concentration de 0,01 à 40% en poids par rapport au poids total de la composition.

5. Composition cosmétique selon l'une quelconque des revendications précédentes, **caractérisée en ce que** la composition est exempte de p-méthylbenzylidénecamphre.

6. Composition cosmétique selon l'une quelconque des revendications précédentes, **caractérisée en ce que** la composition se trouve sous forme d'une émulsion H/E.

7. Composition cosmétique selon la revendication 6, **caractérisée en ce que** la composition contient un ou plusieurs émulsifiants H/E, choisis dans le groupe formé par les composés stéarate-citrate de glycéryle, stéarate de glycéryle (auto-émulsifiant), acide stéarique, sels de stéarate, distéarate de polyglycéryl-3-méthylglucose, Ceteareth-20, stéarate de PEG-40, cétéarylsulfate de sodium.

8. Composition cosmétique selon l'une quelconque des revendications 1-5, **caractérisée en ce que** la composition se trouve sous forme d'une émulsion E/H.

9. Composition cosmétique selon la revendication 8, **caractérisée en ce que** la composition contient un ou plusieurs émulsifiants E/H choisis dans le groupe formé par les composés 2-dipolyhydroxystéarate de polyglycéryle, dipolyhydroxystéarate de PEG-30, Cetyl Dimethicon Copolyol, diisostéarate de polyglycéryle-3.
